# FASCICULE DE BREVET EUROPEEN

(11) **EP 1 151 744 B1**
(45) Date de publication et mention de la délivrance du brevet: **13.09.2006**
(21) Numéro de dépôt: 01401022.7
(22) Date de dépôt: 20.04.2001
(51) Int. Cl.: A61Q 19/00, A61K 8/55

(54) **Composition cosmétique et/ou dermatologique à stabilité améliorée**
Kosmetische und/oder dermatologische Zusammensetzung mit verbesserter Stabilität
Cosmetic and/or dermatologic composition with enhanced stability

(30) Priorité: 05.05.2000 FR 0005805
(43) Date de publication de la demande: 07.11.2001
(73) Titulaire: Laboratoire Nuxe, 75010 Paris (FR)
(72) Inventeur: Leclere, Jacques, 45500 Saint-Gondon (FR)
(74) Mandataire: L'Helgoualch, Jean

(56) Documents cités:
- EP-A- 0 608 989
- WO-A-00/47184
- US-A- 4 482 537
- US-A- 5 759 525
- US-A- 5 849 273

## Description

La présente invention concerne une nouvelle composition cosmétique et/ou dermatologique, et plus particulièrement une nouvelle composition à faible teneur en alcool et à stabilité améliorée, utilisable par voie topique en cosmétologie et/ou en dermatologie pour les soins et l'hydratation de la peau.

La peau comprend des couches superficielles, à savoir l'épiderme, et des couches plus profondes, le derme et l'hypoderme, et chacune possède des propriétés spécifiques permettant à l'ensemble de réagir et s'adapter aux conditions de son environnement. L'épiderme, qui constitue la couche externe, joue un rôle fondamental pour assurer la protection et le maintien d'une bonne trophicité. C'est pourquoi de nombreuses compositions ont été mises au point afin de le protéger et d'améliorer ses fonctions.

Ces compositions cosmétiques et dermatologiques destinées au traitement des affections de la peau par application topique doivent respecter certaines conditions pour être bien acceptées par les utilisateurs. Plus particulièrement, elles doivent posséder de bonnes propriétés physiques, notamment de consistance et d'onctuosité, présenter une efficacité satisfaisante, et être agréables à utiliser. De plus, elles doivent pouvoir être conservées dans des conditions normales de température et d'hygrométrie sans se dégrader sensiblement et en conservant leurs propriétés pendant des périodes prolongées. Il est donc important qu'elles présentent une excellente stabilité dans le temps, dans des conditions normales de conservation.

De nombreux agents hydratants ont été utilisés dans les compositions cosmétiques, et par exemple des compositions sous forme d'émulsion ou de gel contenant de la glycérine (ou glycérol) sont décrites dans les brevets FR-A-2.742.354 et FR-A-2.703.907.

Diverses compositions antisolaires contiennent aussi des agents hydratants, et par exemple le brevet US 5.849.273 décrit une composition contenant un composé anti-UV-A de type dibenzoylméthane, stabilisée au moyen d'un salicylate d'alkyle et comprenant de la glycérine ou un glycol comme agent hydratant. Une autre composition antisolaire est décrite dans le brevet US 5.729.325 où la composition contient un filtre UV-B triazinique associé à la glycérine comme agent hydratant. La glycérine, des glycols et le sorbitol peuvent aussi être utilisés comme agents hydratants dans des compositions de maquillage, comme dans le brevet EP 608.989, en association avec un émulsionnant et un stabilisant cellulosique.

Les lécithines, ou phosphatidyl cholines, sont des phosphoglycérides généralement extraits de l'huile de soja. En raison de leurs propriétés rhéologiques, les lécithines sont souvent utilisées dans des émulsions pour conférer à la composition des propriétés de douceur et d'onctuosité. Ainsi par exemple, le brevet FR 2.777.179 décrit une composition cosmétique pour application topique comprenant une lécithine de soja en association avec de l'eau et un liquide hydrophobe tel qu'un hydrocarbure saturé ou une cyclométhicone, dans des proportions déterminées pour améliorer l'homogénéité des formulations. La demande WO 97.37637 décrit une composition à base de liposomes contenant une lécithine hydrogénée en combinaison avec un alcool (de préférence l'éthanol) destinée à faciliter la pénétration du principe actif (vitamine D) dans la peau.

La présente invention a pour objet une composition comme décrite à la revendication 1.

La présente invention a précisément pour objet une composition cosmétique et/ou dermatologique présentant une excellente stabilité, comprenant en combinaison un ou plusieurs agents hydratants, un ou plusieurs dérivés cellulosiques, un polymère ou copolymère vinylique réticulé, ainsi qu'un solvant à base de glycol.

Enfin, la présente invention a encore pour objet un procédé de traitement cosmétologique de la peau consistant à appliquer sur la peau une composition comme indiqué ci-dessus.

Outre les composants indiqués ci-dessus, la composition conforme à la présente invention contient avantageusement une lécithine hydrogénée.

La lécithine hydrogénée utilisée dans l'invention est de préférence une lécithine de soja hydrogénée ou une lécithine de tournesol hydrogénée. Les lécithines disponibles dans le commerce contiennent généralement de la lécithine pure (phosphatidyl choline) en mélange avec d'autres phosphoglycérides tels que céphaline (en particulier la phosphatidyl éthanolamine) et le phosphatidyl inositol. Conformément à l'invention on peut utiliser la lécithine pure hydrogénée ou une lécithine du commerce hydrogénée. Un exemple de lécithine hydrogénée utilisable dans l'invention est celle vendue dans le commerce sous la marque Emulmetik 320® par la société Lucas Mayer (lécithine de soja hydrogénée).

Suivant une forme préférentielle de réalisation, la composition de l'invention comprend un agent hydratant choisi parmi la glycérine (glycérol), le sorbitol, le maltitol et leurs dérivés, en combinaison avec le dérivé cellulosique et le polymère ou copolymère vinylique réticulé, ainsi que la lécithine hydrogénée et un solvant à base de glycol, en particulier le propylène glycol.

Le dérivé cellulosique peut être plus particulièrement choisi parmi l'hydroxyéthyl cellulose et l'hydroxypropyl cellulose.

Comme indiqué ci-dessus, il est tout particulièrement avantageux d'incorporer dans la composition de l'invention un polymère ou copolymère vinylique réticulé, et par exemple un copolymère à base d'acrylate et d'isodécanoate de vinyle réticulé comme celui disponible dans le commerce sous la marque Stabilene 30® de la société 3V Sigma, en mélange avec de l'hydroxy-éthyl cellulose (Liporamnosan® de la société Vevy). Un tel additif améliore la stabilité de la composition et sa qualité de toucher.

La composition peut aussi avantageusement contenir un mélange complexe de chicorée et d'acide stéarique, par exemple celui disponible dans le commerce sous la marque Acticell® de la société Coletica.

La composition conforme à la présente invention comprend donc en combinaison au moins un agent hydratant tel que le glycérol, un polymère ou un copolymère vinylique réticulé, un dérivé cellulosique tel que l'hydroxy-éthyl ou l'hydroxypropyl cellulose, et un solvant à base de glycol, en association, le cas échéant avec une lécithine hydrogénée.

Les rapports en poids de ces composants sont de 0,2 % à 3 % pour l'agent hydratant, de 0,01 % à 1 % pour le polymère ou le copolymère vinylique réticulé, de 0,05 % à 3 % pour le solvant à base de glycol, et de 0,1 % à 8 % pour la lécithine hydrogénée.

Plus précisément, la teneur en lécithine hydrogénée est généralement comprise entre 0,1 et 8 % en poids par rapport au poids total de la composition, et de préférence entre 0,2 et 5 % en poids.

Les essais effectués avec des compositions conformes à la présente invention ont montré que l'incorporation de la lécithine hydrogénée complète l'action du glycol et améliore sensiblement la stabilité de l'émulsion formée. Bien que le glycol et la lécithine soient tous deux connus en cosmétique et puissent parfois être utilisés dans une même composition, rien ne permettait de prévoir que l'addition d'une lécithine hydrogénée apporterait une amélioration sensible aux propriétés de l'émulsion, dont la stabilité peut en outre être renforcée par la combinaison du copolymère réticulé et du dérivé cellulosique.

Un autre avantage de la composition de la présente invention est qu'elle permet de réduire considérablement la teneur en alcools, en particulier en mono-alcools. Les alcools sont couramment utilisés dans les compositions cosmétiques comme émulsionnants et comme solvants car ils permettent d'obtenir généralement des compositions liquides stables et homogènes. Il est cependant souhaitable d'en réduire la teneur sans perdre les propriétés qu'ils procurent. Or, on a constaté que les compositions conformes à la présente invention possèdent d'excellentes propriétés de stabilité et d'homogénéité malgré une faible teneur en alcools, c'est-à-dire moins de 0,5 % en mono-alcools et moins de 3 % environ en glycols, alors que les compositions usuelles de la technique contiennent bien souvent de l'ordre de 6 à 20% de glycols et au total plus de 20 % d'alcools.

Enfin, on a constaté que l'émulsion de l'invention ayant une composition comme indiqué ci-dessus, de type lamellaire et ne contenant aucun dérivé éthoxylé, procure de très bons résultats d'hydratation de la peau avec une faible quantité d'agent hydratant (moins de 3 % de glycérol). L'absence de dérivé éthoxylé, dont la toxicité est connue, procure à la composition de l'invention l'avantage d'une bonne sécurité d'utilisation.

Les compositions conformes à la présente invention sont destinées à être administrées par voie topique, par exemple par application ou pulvérisation sur la peau, et comprennent donc, outre les composants essentiels précités, divers excipients, additifs et supports usuels acceptables sur le plan dermatologique et cosmétique, choisis en fonction de leurs propriétés connues et de la forme galénique choisie. Ainsi, on peut incorporer dans la composition des conservateurs, des émulsionnants, des épaississants, des gélifiants hydrophiles ou lipophiles, des colorants, des antioxydants, des agents hydratants additionnels, des tensioactifs, des propulseurs pour aérosols, des parfums et divers additifs destinés à améliorer les propriétés physiques de la composition. Il peut aussi être avantageux d'incorporer dans la composition de l'invention, des filtres ou écrans solaires choisis en fonction du degré de protection recherché.

Par exemple, on peut utiliser dans l'invention les conservateurs usuels de la technique des compositions dermatologiques ou cosmétologiques, et par exemple l'acide benzoïque ou un p-hydroxy-benzoate d'alkyle, et en particulier le p-hydroxy-benzoate de méthyle (Méthylparaben) et le p-hydroxy-benzoate de propyle (Propylparaben), isolément ou en combinaison.

Divers émulsionnants additionnels peuvent être choisis parmi ceux couramment employés dans la technique tels qu'un Polysorbate (par exemple le Tween 60® ou le Tween 80®), un ester de sorbitan tel que le stéarate ou le laurate de sorbitan, ou des dérivés d'acide stéarique comme le stéarate de PEG 100®, un stéarate de propylène glycol, un stéarate de polyéthylène glycol, un stéareth ou un cétéareth, ou encore un dérivé de sucrose (par exemple un ester) non éthoxylé. Suivant l'invention, l'émulsionnant additionnel est de préférence un dérivé de sucrose non éthoxylé.

L'agent hydratant ou humectant additionnel peut être par exemple un polyol, le sorbitol, le maltitol, le pentaérythritol, les polyacrylates et polyméthacrylates de glycéryle, outre le glycérol et les dérivés de glycérol précités. On peut aussi ajouter des émollients tels qu'un malate d'alkyle, l'isohexadécane, des triglycérides d'acide caprique ou caprylique, etc.

Les gélifiants ou épaississants peuvent être choisis par exemple parmi les polyacrylamides (par exemple du type Carbopol), les copolymères acrylate/acide acrylique ou acrylamide/acide acrylamido propane sulfonique, les dérivés de cellulose comme l'hydroxypropyl cellulose, ou les gommes naturelles comme la gomme de xanthane.

Il peut aussi être avantageux d'incorporer dans la composition un actif complémentaire destiné à améliorer le comportement de la peau, tel que le tocophérol, la vitamine A (rétinol), l'acide rétinoïque, des agents bactéricides, etc.

Les compositions peuvent se présenter sous forme de crèmes, laits, émulsions huile-dans-eau (H/E) ou émulsions eau-dans-huile (E/H), lotions et émulsions pour pulvérisation, gels, masques ou pommades et de préférence sous forme de lotions ou émulsions sans alcool ou à faible teneur en alcool. Dans le cas de l'application par pulvérisation, les systèmes généralement utilisés sont soit par pompe atmosphérique, soit par poche souple ou par pression de gaz tel que air, azote ou dioxyde de carbone.

Les compositions suivant la présente invention peuvent être utilisées pour divers traitements cosmétologiques ou dermatologiques, en fonction des principes actifs qui y sont incorporés, par exemple pour la prévention et la diminution des rides, pour lutter contre le vieillissement de la peau lié à l'âge ou à l'exposition au soleil, ainsi que pour l'hydratation et la protection de la peau contre les diverses agressions de l'environnement, par exemple les agressions climatiques (vent, froid, pluie, etc.), chimiques (produits de nettoyage et détergents) ou encore la pollution.

Les exemples suivants illustrent l'invention plus en détail sans en limiter la portée. Sauf indication contraire, toutes les parties et pourcentages sont exprimés en poids.

### Exemple 1

On prépare une émulsion applicable par pulvérisation, ayant la composition pondérale suivante.

| | | |
|---|---|---|
| Glycérine | | 1,00 |
| Hydroxyéthyl cellulose | | 0,03 |
| Acrylate / isodécanoate de vinyle (copolymère réticulé) | | 0,05 |
| Propylène glycol | | 1,00 |
| Lécithine hydrogénée | | 1,20 |
| Complexe chicorée / acide stéarique | | 0,50 |
| Phenoxetol | | 0,50 |
| Méthyl- et Propyl-paraben | | 0,30 |
| EDTA trisodique | | 0,10 |
| Dimethicone copolyol | | 1,50 |
| Phényl triméthicone | | 1,00 |
| Alcool cétylique | | 0,25 |
| Acide déhydroacétique | | 0,10 |
| Neopentanoate d'isodécyle | | 1,00 |
| Tocophérol | | 0,20 |
| Laits végétaux | | 1,00 |
| Complexe hydratant | | 7,00 |
| Parfums | | 0,30 |
| Eau | q.s.p. | 100,00 |

L'émulsion dont la composition est donnée ci-dessus peut être préparée par les techniques conventionnelles. Par exemple, la lécithine est tout d'abord hydratée en présence de glycérine pendant environ 20 minutes à une température de 75°C puis la phase lipidique est ajoutée en fin d'hydratation sous agitation en utilisant un agitateur rotatif (500 trs/min) à fort pouvoir de cisaillement pendant environ 20 min. jusqu'à obtention de particules de taille inférieure à 150 nm.

Les gélifiants sont ensuite ajoutés, puis le copolymère est neutralisé, le mélange est refroidi à environ 30°C, et on ajoute les principes actifs et les parfums. L'addition du mélange d'hydroxyéthyl cellulose et de copolymère réticulé neutralisé en fin de fabrication a pour effet de stabiliser la dispersion sans augmenter sensiblement la viscosité et en conservant une structure suffisamment thixotropique pour permettre l'application par pulvérisation.

### Exemple 2

Par la même méthode que dans l'Exemple 1, on prépare une émulsion pulvérisable antirides ayant la composition pondérale suivante.

| | | |
|---|---|---|
| Glycérine | | 1,50. |
| Hydroxyéthyl cellulose | | 0,07 |
| Acrylate / isodécanoate de vinyle (copolymère réticulé) | | 0,07 |
| Propylène glycol | | 1,50 |
| Lécithine hydrogénée | | 2,00 |
| Complexe chicorée / acide stéarique | | 1,00 |
| Phenoxetol | | 0,50 |
| Méthyl- et Propyl-paraben | | 0,30 |
| EDTA trisodique | | 0,10 |
| Acide déhydroacétique | | 0,10 |
| Phényl triméthicone | | 2,00 |
| Alcool cétylique | | 0,20 |
| Chlorphénésion | | 0,05 |
| Isononanoate d'isononyle | | 1,00 |
| Tocophérol | | 0,25 |
| Tromethamine | | 0,08 |
| Hydrolysat de protéines de soja | | 5,00 |
| Mucilages de Cassia Angustifolia | | 3,00 |
| Parfums | | 0,10 |
| Eau | q.s.p. | 100,00 |

### Exemple 3

Par la même méthode que dans l'Exemple 1, on prépare une émulsion pulvérisable hydratante et autobronzante ayant la composition pondérale suivante.

| | | |
|---|---|---|
| Glycérine | | 2,00 |
| Hydroxyéthyl cellulose | | 0,05 |
| Acrylate / isodécanoate de vinyle (copolymère réticulé) | | 0,10 |
| Propylène glycol | | 1,00 |
| Lécithine hydrogénée (Emulmetik 320®) | | 2,00 |
| Complexe protéines de soja/acide stéarique | | 0,50 |
| Sclerotium Gum | | 0,10 |
| Phenoxetol | | 0,50 |
| Méthyl- et Propyl-paraben | | 0,30. |
| EDTA trisodique | | 0,10 |
| Acide déhydroacétique | | 0,10 |
| Dimethicone | | 1,00 |
| Phényl triméthicone | | 1,00 |
| Palmitate de cétyle | | 0,15 |
| Neopentanoate d'isodécyle | | 1,00 |
| Esters de jojoba | | 1,00 |
| Tocophérol | | 0,20 |
| Tromethamin (trisamino) | | 0,10 |
| Erythrulose | | 2,00 |
| Hyaluronate de sodium | | 0,01 |
| Parfums | | 0,10 |
| Eau | q.s.p. | 100,00 |

## Revendications

1. Composition cosmétique et/ou dermatologique à stabilité améliorée et à faible teneur en alcool, **caractérisée en ce qu'**elle comprend en combinaison un ou plusieurs agents hydratants, un ou plusieurs dérivés cellulosiques, un polymère ou copolymère vinylique réticulé, un solvant à base de glycol dans une teneur par rapport au poids total de la composition inférieure à 3% et une lécithine hydrogénée.

2. Composition selon la revendication 1, **caractérisée en ce que** le copolymère vinylique est un copolymère à base d'acrylate et d'isodécanoate de vinyle réticulé.

3. Composition selon la revendication 1, **caractérisée en ce que** l'agent hydratant est choisi parmi la glycérine, le sorbitol, le maltitol et leurs dérivés.

4. Composition selon la revendication 1, **caractérisée en ce que** le dérivé cellulosique est choisi parmi l'hydroxyéthyl cellulose et l'hydroxypropyl cellulose.

5. Composition selon la revendication 1, **caractérisée en ce que** le solvant est un glycol, notamment le propylène glycol.

6. Composition selon la revendication 1, **caractérisée en ce que** la lécithine est une lécithine de soja hydrogénée ou une lécithine de tournesol hydrogénée.

7. Composition selon l'une quelconque des revendications 1 et 6, **caractérisée en ce que** la teneur en lécithine hydrogénée par rapport au poids total de la composition est comprise entre 0,1 % et a %.

8. Composition selon l'une quelconque des revendications 1 et 3, **caractérisée en ce que** la teneur en agent hydratant est comprise entre 0,2 % et 5 %.

9. Composition selon l'une quelconque des revendications 1 et 2, **caractérisée en ce que** la teneur en polymère ou copolymère vinylique réticulé est comprise entre 0,01 % et 1 %.

10. Composition selon l'une quelconque des revendications 1 et 5, **caractérisée en ce que** la teneur en glycol est comprise entre 0,05 % et 3 %.

11. Procédé de traitement cosmétologique de la peau **caractérisé en ce qu'**il consiste à appliquer sur la peau une composition selon l'une quelconque des revendications 1 à 10.

## Claims

1. Cosmetic and/or dermatological composition with improved stability and low alcohol content, **characterized in that** it comprises a combination of one or more moisturizing agents, one or more cellulose derivatives, a crosslinked vinyl polymer or copolymer, a glycol based solvent with a weight ratio lower than 3% of the total weight of the composition, and a hydrogenated lecithin.

2. A composition according to claim 1, **characterized in that** the vinyl copolymer is a crosslinked vinyl acrylate and isodecanoate copolymer.

3. A composition according to claim 1, **characterized in that** the moisturizing agent is selected from glycerin, sorbitol, maltitol and the derivatives thereof.

4. A composition according to claim 1, **characterized in that** the cellulose derivative is selected from hydroxyethyl cellulose and hydroxypropyl cellulose.

5. A composition according to claim 1, **characterized in that** the solvent is a glycol, more particularly propylene glycol.

6. A composition according to claim 1, **characterized in that** the lecithin is a hydrogenated soybean lecithin or a hydrogenated sunflower lecithin.

7. A composition according to any of claims 1 to 6, **characterized in that** the hydrogenated lecithin content is between 0,1% and 8% by weight relative to the total weight of the composition.

8. A composition according to any of claims 1 and 3, **characterized in that** the moisturizing agent content is between 0,2% and 5%.

9. A composition according to any of claims 1 and 2, **characterized in that** the crosslinked vinyl polymer or copolymer content is between 0,01% and 1%.

10. A composition according to any of claims 1 and 5, **characterized in that** the glycol content is between 0,05% and 3%.

11. A method for the cosmetologic treatment of the skin, **characterized in that** it consists in applying onto the skin a composition according to any of claims 1 to 10.

## Patentansprüche

1. Kosmetische und/oder dermatologische Zusammensetzung mit verbesserter Stabilität und geringem Alkoholgehalt, **dadurch gekennzeichnet, dass** sie, in Kombination, ein oder mehrere hydratisierende Mittel, ein oder mehrere Cellulosederivate, ein vernetztes Vinylpolymer oder -copolymer, ein Lösungsmittel auf Glykolbasis in einem auf das Gesamtgewicht der Zusammensetzung bezogenen Gehalt von unter 3 % sowie ein hydriertes Lecithin umfasst.

2. Zusammensetzung nach Anspruch 1, **dadurch gekennzeichnet, dass** das Vinylcopolymer ein Copolymer auf der Basis von Acrylat und vernetztem Vinylisodecanat ist.

3. Zusammensetzung nach Anspruch 1, **dadurch gekennzeichnet, dass** das hydratisierende Mittel aus Glycerin, Sorbit, Maltit und deren Derivaten ausgewählt ist.

4. Zusammensetzung nach Anspruch 1, **dadurch gekennzeichnet, dass** das Cellulosederivat aus Hydroxyethylcellulose und Hydroxypropylcellulose ausgewählt ist.

5. Zusammensetzung nach Anspruch 1, **dadurch gekennzeichnet, dass** das Lösungsmittel ein Glykol, insbesondere Propylenglykol, ist.

6. Zusammensetzung nach Anspruch 1, **dadurch gekennzeichnet, dass** das Lecithin ein hydriertes Sojalecithin oder ein hydriertes Sonnenblumenlecithin ist.

7. Zusammensetzung nach einem der Ansprüche 1 und 6, **dadurch gekennzeichnet, dass** der Gehalt an hydriertem Lecithin, bezogen auf das Gesamtgewicht der Zusammensetzung, im Bereich von 0,1 % bis 8 % liegt.

8. Zusammensetzung nach einem der Ansprüche 1 und 3, **dadurch gekennzeichnet, dass** der Gehalt an hydratisierendem Mittel im Bereich von 0,2 % bis 5 % liegt.

9. Zusammensetzung nach einem der Ansprüche 1 und 2, **dadurch gekennzeichnet, dass** der Gehalt an vernetztem Vinylpolymer oder -copolymer im Bereich von 0,01 % und 1 % liegt.

10. Zusammensetzung nach einem der Ansprüche 1 und 5, **dadurch gekennzeichnet, dass** der Gehalt an Glykol im Bereich von 0,05 % bis 3 % liegt.

11. Kosmetisches Behandlungsverfahren für die Haut, **dadurch gekennzeichnet, dass** es darin besteht, eine Zusammensetzung nach einem der Ansprüche 1 bis 10 auf die Haut aufzutragen.
